# EUROPEAN PATENT SPECIFICATION

(11) **EP 0 286 743 B1**
(45) Date of publication and mention of the grant of the patent: **09.03.1994**
(21) Application number: 87303248.6
(22) Date of filing: 14.04.1987
(51) Int. Cl.: A61B 10/00, G01N 11/00

(54) **Process and apparatus for determining female ovulation time by measurement of saliva viscoelasticity**
Verfahren und Vorrichtung zum Erkennen des Zeitpunkts des Eisprungs bei Frauen durch Messen der Viscoelastizität vom Speichel
Procédé et dispositif pour déterminer la période de l'ovulation féminine par mesure de la visco-élasticité de la salive

(43) Date of publication of application: 19.10.1988
(73) Proprietor: The Academy of Applied Science Inc., Concord New Hampshire 03301 (US)
(72) Inventor: Kosasky, Harold J., Massachusetts 02167 (US)
(74) Representative: Allsop, John Rowland

(56) References cited:
- US-A- 4 059 986
- US-A- 4 072 045
- US-A- 4 164 212
- US-A- 4 570 478

## Description

The present invention relates to the determination of the time of ovulation through the monitoring of the viscoelasticity or tackiness of the female saliva; being also useful for determination of hormonal and related functions associated with ovulation, and conversely with similarly determining the non-ovulation effectiveness of oral contraceptives and the like.

It has earlier been known that the cervical mucus of a female has a minimum thinness or highest fluidity just before ovulation and closely coincident with the surge in estradiol and in the luteinizing hormone (LH) peak -- a phenomenon that led to my previous activities in the development of techniques for monitoring the viscoelasticity and other properties of cervical mucus as a predictor of time of ovulation, and the improvement in rheometer or viscometer apparatus for measuring such viscoelastic properties ( L. E. Kopito and H.J. Kosasky, "The tackiness rheometer determination of the viscoelasticity of cervical mucus", Human Ovulation, edited by E.S.E. Hafez, Elsevier, North-Holland Biomedical Press, 1979, commencing with p. 351, on; and U.S. Letters Patent No. 4,002,056). Through the viscoelasticity of the cervical mucus has several small dips in its characteristic curve of viscosity versus time preceding, during and following ovulation (a four-day period), there is a distinct identifiable minimum coincident with the peaking of estradiol, representing the thinning of bodily secretions. Instruments designed to measure this effect are described in, for example, said Letters Patent and in U.S. Letters Patent No. 4,072,045.

While this is a most desirable technique for monitoring ovulation times and related hormonal and rheologic property functions, it requires the insertion of cervical mucus sampling applicators into the vagina, which is an impediment to universal usage, has features of discomfort, demands sanitation, and requires proper positioning at the central portion of the cervix. The non-observable aspiration or extraction of the hoped-for cervical mucus, moreover, can result in the withdrawal of undesired vaginal fluids that can upset reliable measurements. Additionally, the mucus is of relatively high viscosity (5000-50,000 centipoise), requiring one-use sampling surfaces in viscometers used to measure the viscoelasticity of the mucus.

Saliva and other body fluids have been known to undergo chemical changes during the menstrual cycle (for example, G. Oster, et al, "Cyclic variation of sialic acid content in saliva", Am. J. Obstet Gynercol, Vol. 114, No. 2, p. 190, Sept. 15, 1972, and I. Shannon, "Composition and Secreta of Saliva", 1974. Until the discovery underlying the present invention, however, the unique reproducible and single minimum viscosity dip characteristic of saliva during the ovulation cycle was apparently not detected -- the saliva cycle, indeed, being restricted to about two days as compared with four days for cervical mucus, and having a viscosity range but 1/10 to 1/100 of that of cervical mucus, and thus understandably difficult to detect, particularly with cervical mucus instruments.

In US 4 146 212 there is disclosed an instrument for obtaining and testing cervical and oral mucus for determining the rheological properties thereof in order to predict and indicate the inception and the presence of ovulation for conception control.

I have now discovered, indeed, that particularly sublingual saliva (which has been noted to have less water than saliva from other parts of the mouth, and about 50% mucus, as contrasted with over 70-90% water in submandibular saliva) produces a unique and reliably measurable minimum dip in viscoelasticity or tackiness that is coincident with the ovulation cycle and its surge of estradiol, and also tracks the minimum dip in cervical mucus viscoelasticity, though it is much narrower (48 hours, vs 4 days) and far below the viscosity levels of such mucus, as before stated. The use of saliva, furthermore, totally obviates the difficulties and disadvantages associated with the extraction of cervical mucus from the vagina and thus promises to be widely and universally acceptable as an ovulation monitor, and most simply usable by even the less educated, as well.

An object of the present invention, accordingly, is to provide a new and improved process for determining the time of ovulation and related hormone functions of a female that, though based on female fluid viscosity measurements, is not subject to the before-described and other limitations of cervical mucus viscoelasticity measurements, nor the more undesirable blood hormone measurements or other chemical measurements, but employs a simpler and distinctly unique minimum saliva tackiness phenomenon as a much improved alternative.

According to the present invention there is provided a process for determining the time of ovulation of a female comprising sublingually extracting a sample of saliva from the mount of a female; compressing the sample between opposing surfaces to cause adherence of the saliva to the same; applying a predetermined relative pulling or shearing force between said surfaces correspondingly to stretch the saliva, as a measure of its degree of viscoelasticity under such predetermined force; comparing the measured degree of viscoelasticity with a predetermined unique monthly minimum dip of viscoelasticity in the saliva of the female; and indicating substantial coincidence of the measured degree of viscoelasticity with said minimum dip to identify that the time of mid-cycle of ovulation is about to occur.

The embodiments of the present invention will now be described by way of example with reference to the accompanying drawings wherein;
Fig 1 is a graph (viscosity plotted in units of centipoise along the ordinate, being plotted logarithmically for cervical mucus and linearly for saliva, and as a function of time in days plotted along the abscissa) showing the phenomenon of coincidence of a unique sublingual saliva viscosity minimum with proximal ovulation, employed in the process of the invention, and compared with a similar phenomenon with cervical mucus, but occurring over a much narrower time span and over a small fraction of the viscosity range involved with cervical mucus;
Fig 2 is an enlarged graph of the saliva characteristic of Fig 1;
Fig 3A is a longitudinal section of a simple ovulation monitor instrument for using the technique of the invention; and
Fig. 3B is a similar fragmentary view of a modification.

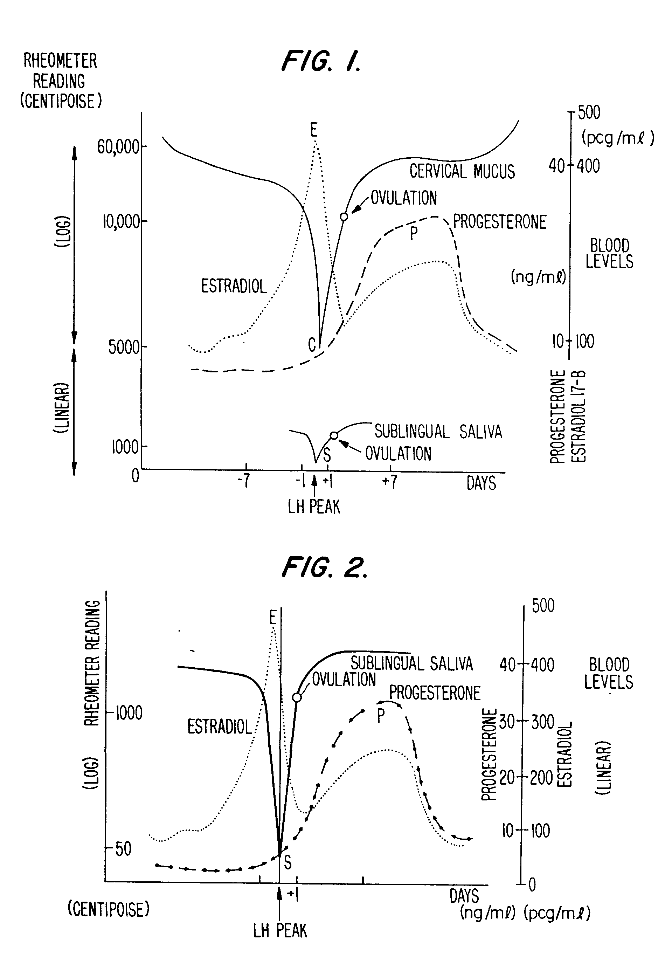

Referring to Fig. 1, underlying the invention, as before stated, is the discovery that quantitatively, reliably and reproducably, the viscoelasticity of particularly sublingual saliva goes through a unique minimum S (bottom graph) at the mid-cycle, coincident with the peaking surge in estradiol E, and somewhat paralleling the cervical mucus dip C, (upper plot) though with the saliva being relatively watery (30 to 1500 centipoise) as compared with the 5000-50,000 centipoise viscosity range of cervical mucus.

The coincidence with the peak of luteinizing hormone "LH Peak" and the following progesterone post-ovulation development P is also shown in Fig. 1 (plotted along the right-hand ordinate in units of ng/ml).

As shown in Fig. 1 and as previously delineated, the cervical mucus viscoelastic characteristic curve dip and rise extends over a period of about 4 days, with the actual ovulation occurring within a day and a half of the minimum dip C. The sublingual saliva characteristic falls much more steeply and extends over a period of about 48 hours, with ovulation occurring within 24 hours of the minimum S. The measurements of the cervical mucus were taken with the rheometer equipment described in my said Human Ovulation article; and the saliva measurements were made with a modified rotary type eccentric glass cylinder rheometer having randomly roughened opposing surfaces (of the order of 10-20 thousandths of an inch) and with saliva samples of about 0.25 mm in thickness.

In the normal ovulating woman, typical cervical mucus confirmatory patterns C have been observed, with viscoelasticity beginning to decrease, Fig. 1, three days before the LH peak to minimal values C. Within one to two days after the LH peak, the viscosity has risen to the pre-ovulatory levels, but then may dip again, as shown to the right, but not as low as the minimum C. The decreasing viscoelasticity in saliva, as before stated, has been found to have a shorter or narrower time span and usually without subsequent dips (or earlier dips) as may occur with cervical mucus. The minimum thinness at S represents the time of maximum sperm penetration, so that advent of the commencement of the saliva viscoelasticity dip, signals the approach of an appropriate time for conception. This coincidence with mid-cycle, moreover, has been confirmed with blood tests, the levels of which are calibrated along the right-hand ordinate (pcg/ml), Figs. 1 and 2.

The viscoelasticity of the saliva thus correlates with the hormonal relationship of the menstrual cycle, with midcycle and the LH peak, and then rises to the pre-ovulatory levels.

Sublingual saliva was examined 1295 times in 73 patients through 206 cycles. 831 samples of cervical mucus and sublingual saliva were each obtained daily from 25 women in 112 cycles, who were taking oral progestogens. In 60% of the subjects, simultaneous blood hormone assays were also performed. The results showed that the viscoelasticity of sublingual saliva parallels that of cervical mucus, in all cases; with the highest fluidity occurring at midcycle at the time of the estradiol surge. In the ovulating woman, typical confirmatory patterns are seen in all of the measurements.

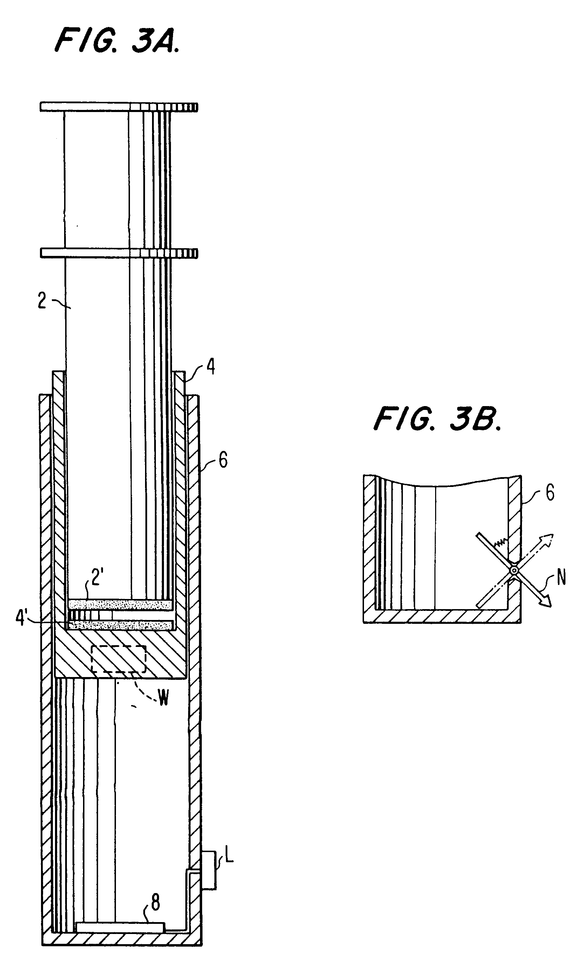

A very simple instrument for universal and inexpensive use, including adaptability for throw away after a use if desired, is shown in Fig. 3A upon a somewhat enlarged scale -- the instrument being constructable in dimensions commensurate with a narrow syringe. The plunger 2, such as a glass piston, or other sample carrier, is provided with a saliva sample surface 2', roughened as before discussed finely enough to cause adhering of the saliva when the plunger 2 is removed and placed under the tongue to extract a sample (much as in using oral thermometers). The plunger is then inserted, as shown, within a recessed or re-entrant coaxial cylindrical tube 4, the outer walls of which are fitted within an outer coaxial cylinder 6 and with either a fractional fit or a detent or the like holding the tube 4 in elevated position, as shown, in the outer cylinder 6. At the bottom of the recess of the cylinder is an opposing roughened surface 4' similar to the surface 2ʹ and between which and the surface 2ʹ the saliva sample is compressed when the plunger 2 is inserted down into the recess of the cylinder 4. Fastened to the bottom of the cylinder 4 is a predetermined weight W (shown embedded in the cylinder base) adjusted to be sufficient to cause the cylinder 4 to separate from the plunger 2 and to drop, under the influence of gravity, to the bottom of the outer cylinder 6 in the event that the viscoelasticity of the saliva sample is not sufficient to hold the cylinder 4 elevated, and to prevent fracture under the pulling or stretching force of the weight (as when the viscoelasticty dips toward S of Fig. 1). The weight may be of the order of 5 grams, calibrated by the physician.

The striking of the bottom of the cylinder 4 against an electrical contact or switch 8 at the base of the outer cylinder 6 may complete a circuit containing a miniature battery and control the activatation of an indicator lamp L carried by the cylinder 6. If the light goes on, this is an indication to the woman of the near or substantial coincidence of the thusly measured degree of saliva viscosity with the time of the dipping characteristic; and she can take advantage of the up-coming ovulation for fertilization or the converse.

Alternatively, a less expensive pivotable spring-retained needle indicator N may be provided, as shown in Fig. 3B, as may other indicating devices be employed as well.

For non-ovulating cycles, as in the case of women taking birth control pills, the salivary viscoelasticity does not show this characteristic ovulatory deep dip S, but rather remains elevated throughout the cycle.

The invention thus enables an immediate and reliable assessment of the ovulatory and concomitant hormonal status of the patient as determined in her saliva, without the necessity for vaginal intrusion or blood sampling.

## Claims

1. A process for determining the time of ovulation of a female comprising sublingually extracting a sample of saliva from the mouth of a female; compressing the sample between opposing surfaces to cause adherence of the saliva to the same; applying a predetermined relative pulling or shearing force between said surfaces correspondingly to stretch the saliva, as a measure of its degree of viscoelasticity under such predetermined force; comparing the measured degree of viscoelasticity with a predetermined unique monthly minimum dip of viscoelasticity in the saliva of the female; and indicating substantial coincidence of the measured degree of viscoelasticity with said minimum dip to identify that the time of mid-cycle of ovulation is about to occur.

2. A process as claimed in claim 1 wherein the saliva of the female is calibrated to determine said minimum dip of viscoelasticity and said predetermined force is adjusted to cause fracture of the sample at or substantially at said minimum dip.

3. A process as claimed in claim 1 or 2 wherein the said sample is of the order of 0.23 mm in thickness.

4. Ovulation indicating apparatus comprising a plunger (2) provided with an end surface (2') insertable in the mouth to coat the surface with an adhering saliva sample, a re-entrant tube (4) for receiving the plunger (2) and having an opposing bottom surface (4') against which the sample carried by the end surface (2') of the plunger (2) is compressed, force applying means (W) carried by the tube (4) for applying a pulling or stretching force between the surfaces (2'), (4') the force being adjusted to cause fracture of the saliva sample and separation of the plunger (2) and the tube (4) when the viscoelasticity of the saliva is at or substantially at a minimum value prior to ovulation, and indicating means (6, 8, 6) controlled by said separation for indicating such fracture to enable intercourse abstinence or activity.

5. Ovulation indicating apparatus in which the force applying means is a calibrated weight (W).

## Patentansprüche

1. Verfahren für die Erkennung des Zeitpunkts des Eisprungs bei Frauen, bei dem
eine Speichelprobe sublingual aus dem Mund einer Frau entnommen wird;
die Probe zwischen zwei einander gegenüberliegende Flächen gepreßt wird, um ein Anhaften des Speichels an diesen zu bewirken;
eine vorbestimmte, relative Zug- oder Scherkraft zwischen den Flächen ausgeübt wird, um den Speichel zu strecken bzw. zu dehnen, und zwar als eine Messung des Maßes seiner Viskoelastizität unter der vorbestimmten Kraft;
der gemessene Wert der Viskoelastizität mit einem individuellen bzw. typischen, vorbestimmten monatlichen Minimum bzw. Einbruch der Viskoelastizität in dem Speichel der Frau verglichen wird, und
die im wesentliche Deckung bzw. Koinzidenz des gemessenen Wertes der Viskoelastizität mit dem Minimum angezeigt wird, um festzustellen, daß der Eintritt des Zeitpunkts des Einsprungs bevorsteht.

2. Verfahren nach Anspruch 1, bei dem der Speichel der Frau kalibriert wird, um den minimalen Wert der Viskoelastizität festzustellen und bei dem die vorgegebene Kraft so eingestellt ist, daß eine Franktur bzw. ein Reißen der Probe bei dem Minimalwert oder im wesentlichen bei dem Minimalwert eintritt.

3. Verfahren nach Anspruch 1 oder 2, dadurch gekennzeichnet, daß die Probe eine Dicke in der Größenordnung von 0,23 mm aufweist.

4. Vorrichtung zum Erkennen oder Anzeigen,
mit einem Plunger (2) mit einer Stirnfläche (2'), die in den Mund einführbar ist, um die Strinfläche mit einer anhaftenden Speichelprobe zu bedecken,
mit einem bei der Ausnehmung bildenden Rohr (4), das zur Aufnahme des Stößels (2) dient und eine gegenüberliegende Bodenfläche (4') aufweist, gegen die die an der Stirnfläche (2') des Stößels (2) gehaltende Probe anpreßbar ist,
mit Mittel (W) zum Erzeugen einer Kraft, die an dem Rohr (4) vorgesehen sind, um eine Zug- oder Streckkraft zwischen den Flächen (2'), (4') zu erzeugen, wobei die Kraft so eingestellt ist, um ein Reißen der Speichelprobe und ein Trennen des Stößels (2) und des Rohres (4) zu verursachen, wenn die Viskoelastizität des Speichels dem Minimalwert oder nahezu dem Minimalwert vor dem Eisprung entspricht, sowie
mit Anzeigemitteln (6, 8, 6), die durch das Trennen gesteuert sind und dieses Trennen bzw. Abreißen anzeigen, um Abstinenz oder Aktivität beim geschlechtlichen Verkehr zu ermöglichen.

5. Vorrichtung zum Erkennen bei der die Mitteln zum Aufbringen der Kraft ein geeichtes Gewicht (W) sind.

## Revendications

1. Procédé pour déterminer le moment de l'ovulation d'une femelle comprenant l'extraction sublinguale d'un échantillon de salive de la bouche d'une femelle ; la compression de l'échantillon entre des surfaces opposées pour provoquer l'adhérence de la salive à celles-ci ; l'application d'une force de traction ou de cisaillement déterminée à l'avance entre ces surfaces pour étirer la salive en conséquence, comme mesure de son degré de viscoélasticité sous l'action de cette force déterminée à l'avance ; la comparaison du degré de viscoélasticité mesuré avec un abaissement minimum mensuel unique déterminé à l'avance, et la constatation d'une coïncidence notable du degré de viscoélasticité mesuré avec cet abaissement minimum, ce qui indique que le temps du milieu du cycle d'ovulation est proche.

2. Procédé selon la revendication 1, dans lequel la salive de la femelle est étalonnée pour déterminer cet abaissement minimum de la viscoélasticité et dans lequel cette force déterminée à l'avance est ajustée pour provoquer la rupture de l'échantillon au moment, ou pratiquement au moment, de cet abaissement.

3. Procédé selon la revendication 1 ou 2, dans lequel cet échantillon a une épaisseur de l'ordre de 0,23 mm.

4. Appareil indiquant l'ovulation comprenant un piston (2) muni d'une surface d'extrémité (2') insérable dans la bouche pour revêtir la surface avec un échantillon de salive adhérent, un tube rentrant (4) pour recevoir le piston (2) et ayant une surface inférieure opposée (4') contre laquelle est comprimé l'échantillon porté par la surface d'extrémité (2') du piston (2), des moyens d'application d'une force (W) portés par le tube (4) pour appliquer une force de traction ou de cisaillement entre les surfaces (2'), (4'), la force étant ajustée pour provoquer la rupture de l'échantillon de salive et la séparation du piston (2) et du tube (4) lorsque la viscoélasticité de la salive a une valeur minimale ou pratiquement minimale avant l'ovulation, et des moyens indicateurs (6, 8, 6) commandés par cette séparation, pour indiquer cette rupture, afin de permettre l'abstinence ou l'activité sexuelles.

5. Appareil indiquant l'ovulation, dans lequel le moyen d'application de la force est un poids calibré (W).
